## Europäisches Patentamt

## European Patent Office

## Office européen des brevets

(19)

(11) Veröffentlichungsnummer: **0 039 434**
**B1**

(12) **EUROPÄISCHE PATENTSCHRIFT**

(45) Veröffentlichungstag der Patentschrift:
**11.07.84**

(21) Anmeldenummer: **81102918.0**

(22) Anmeldetag: **15.04.81**

(51) Int. Cl.³: **A 61 B 10/00, G 01 S 7/62,**
**G 01 S 15/89**

(54) **Vorrichtung zum Speichern von Signalen.**

(30) Priorität: **02.05.80 DE 3017027**

(43) Veröffentlichungstag der Anmeldung:
**11.11.81 Patentblatt 81/45**

(45) Bekanntmachung des Hinweises auf die Patenterteilung:
**11.07.84 Patentblatt 84/28**

(84) Benannte Vertragsstaaten:
**AT CH DE FR GB LI NL**

(73) Patentinhaber: **SIEMENS AKTIENGESELLSCHAFT,**
**Berlin und München Wittelsbacherplatz 2,**
**D-8000 München 2 (DE)**

(72) Erfinder: **Buchner, Klaus, Höhenröthstrasse 9,**
**D-8524 Kleinsendelbach (DE)**
Erfinder: **Saugeon, Ulrich, Dipl.-Ing., Isarstrasse 2,**
**D-8520 Erlangen (DE)**

(56) Entgegenhaltungen:
**EP - A - 0 002 061**
**EP - A - 0 005 432**
**DE - A - 2 912 410**
**US - A - 3 800 289**
**US - A - 3 995 253**
**US - A - 4 111 055**

**JEE, JOURNAL OF ELECTRONIC ENGINEERING, Band 16, Nr. 154, Oktober 1979, Seiten 66-69 Tokyo, JP. "A real time ultrasonic diagnostic system for simultaneous image displays"**

## Beschreibung

Die Erfindung bezieht sich auf eine Vorrichtung zum Speichern von digitalen elektrischen Signalen, die in Signalzeilen aufeinanderfolgen, insbesondere von Ultraschallsignalen, mit einem Signalspeicher mit Signaleingängen zum Einspeichern der Signale und Signalausgängen zum Auslesen der gespeicherten Signale, vorzugsweise zum Auslesen in ein Aufzeichnungsgerät, wie ein Bildgerät, wobei der Signalspeicher in eine Anzahl von Speicherabschnitten unterteilt ist.

Unter den Begriff Signale fallen dabei im weitesten Sinne beliebige Signale, wie z.B. physiologische Signale (EKG), Röntgensignale und insbesondere Ultraschallsignale der Medizin oder gleichartige Signale der allgemeinen Technik.

Es sind bereits eine ganze Reihe von Vorrichtungen dieser Art speziell aus der Ultraschall-Bild-Technik bekannt (z.B. DE-OS 2 619 684, DE-AS 2 417 946, US-PS 4 121 250 oder Aufsatz «A Real-Time Ultrasonic Diagnostic System for Dynamic and Still Images: Wireless Echovision» von K.I.Ito et al aus der Zeitschrift JEE, Dezember 1977, Seiten 20 bis 26). Diese Vorrichtungen arbeiten mit einem Signalspeicher, der in herkömmlicher Weise als Orthogonal-Vollbildspeicher aufgebaut ist. Signifikante Eigenschaften eines solchen Orthogonal-Speichers ist, dass die anfallenden Signale seriell in den Speicher eingelesen und anschliessend orthogonal zur Einleserichtung wieder ausgelesen werden. Das serielle Einlesen führt jedoch erfahrungsgemäss zu relativ langen Bildaufbauzeiten, so dass sich ein Konversionsmodus vom langsamen Ultraschallbild zum rascheren Sichtbild ergibt, der nicht hinreichend schnell ist und somit zum Bildflackern führt. Darüber hinaus verlangt der Orthogonal-Speicher herkömmlicher Art auch noch ein relativ starres Adressierschema beim relativ langsamen Einschreiben. Diese Art Speicher ist also hinsichtlich einer erwünschten hohen Variabilität des Einsatzes nicht flexibel genug.

Aus der EP-A-0 002 061 ist die eingangs genannte Vorrichtung bekannt. Wie sich aus Fig. 5 dieser Literaturstelle ergibt, werden Echosignale einer Ultraschalleinrichtung über eine gemeinsame Leitung einem Signalspeicher zugeführt, der aus vier Teilspeichern oder Speicherabschnitten besteht. Diese vier Speicherabschnitte werden gemeinsam von einem ROM gesteuert. Die Datenaufnahme der Echosignale erfolgt dabei so, dass der erste Speicherabschnitt die Echosignale unverzögert zugeführt erhält, während die drei weiteren Speicherabschnitte dieselben Signale über Verzögerungsglieder mit unterschiedlicher Verzögerung zugeleitet bekommen. Das Auslesen erfolgt über vier Verschieberegister mit Hilfe eines Multiplexers in Richtung auf ein Fernsehgerät. Bei dieser Vorrichtung ist wegen des verzögerten Einlesens kein Zeitgewinn zu verzeichnen. Ein orthogonales Auslesen ist nicht vorgesehen.

Aufgabe vorliegender Erfindung ist es, eine Vorrichtung der eingangs genannten Art anzugeben, die mit einem rascher arbeitenden Signalspeicher versehen ist, der zugleich hohe Flexibilität in der Adressierung ermöglicht.

Diese Aufgabe wird erfindungsgemäss dadurch gelöst, dass ein Zeilenspeicher vorgesehen ist, in den die Signalzeilen seriell eingelesen werden, in dem jede Signalzeile in Signalabschnitte aufgeteilt wird und aus dem diese Signalabschnitte parallel zueinander über einen Registermultiplexer zum Signalspeicher ausgelesen werden, so dass die an den Signaleingängen des Signalspeichers anfallenden Signale in die erwähnten Signalabschnitte aufgeteilt sind, dass die zeitlich nacheinander angefallenen Signalabschnitte jeweils in Parallelformation in den Speicherabschnitten des Signalspeichers einspeicherbar sind, wobei die Daten jedes Signalabschnitts seriell in den zugeordneten Speicherabschnitt eingelesen werden, und dass die Speicherabschnitte zeitlich nacheinander orthogonal auslesbar sind in einer solchen Reihenfolge, dass sich nach Zusammensetzung wieder die aus den Signalabschnitten in richtiger Reihenfolge zusammengesetzten Signalzeilen ergeben.

Demnach sind die anfallenden Signale in Abschnitte aufgeteilt, welche wiederum in Parallelformation in den eigentlichen Signalspeicher eingespeichert werden. Diese Parallelübernahme verkürzt die Einschreibzeit, wodurch sich auch die Bildaufbauzeit verkürzt. Das Bildflackern wird dadurch weitgehend vermieden. Der Signalspeicher ist hinsichtlich der Adressierung beliebig organisierbar. Ein starres Adressierschema, wie es bei herkömmlichen Orthogonal-Speichern der Fall ist, wird also vermieden, so dass der Signalspeicher insgesamt auch vielseitiger anwendbar wird.

In vorteilhafter Ausgestaltung der Erfindung sind im Signalspeicher die Speicherabschnitte zeitlich aufeinanderfolgender Signale kreuzweise parallel ineinandergeschaltet in der Weise, dass bei Orthogonal-Auslesung entsprechend der Reihenfolge des Anfallens von Signalabschnitten und bei der Zusammensetzung deren zeitgerechte Aneinanderreihung zu den Formen der Ursprungssignale von selbst erfolgt. Die spezielle Art der kreuzweisen Verschachtelung von Signalabschnitten im Signalspeicher erlaubt ein etwa viermal rascheres Einschreiben von Signalinformation in den Speicher. Dieser Gewinn um den Zeitfaktor vier macht also eine besonders schnelle real-time-Umsetzung möglich.

In weiterer vorteilhafter Ausbildung der Erfindung sollte das Einschreiben und Auslesen von Signalabschnitten in programmierbarer Formation erfolgen. Hierbei sollte insbesondere der Auslesevorgang so programmierbar sein, dass bestimmte Signalabschnitte zu voneinander unterschiedlichen Darstellungsmodes auslesbar sind. Der Signalspeicher kann dabei insbesondere ein Ultraschall-Bildspeicher sein, der so programmierbar ist, dass ein einzelnes Ultraschallbild oder mehrere Ultraschallbilder einspeicherbar sind. Bei mehreren Ultraschallbildern sollte die Auslesung nach Programm so erfolgen, dass die Bilder in beliebiger Konfiguration gleichzeitig an einem Bildgerät, insbesondere Kathodenstrahlröhre, darstellbar sind. Eine derartige Darstellform ist mit herkömmlichen Orthogonal-Speichern bei der erwünscht hohen Bildfrequenz nicht möglich.

Weitere Vorteile und Einzelheiten der Erfindung ergeben sich aus der nachfolgenden Beschreibung eines Ausführungsbeispiels anhand der Zeichnung in Verbindung mit den restlichen Unteransprüchen.

Es zeigen:

Fig. 1 die Anwendung der Erfindung bei Ultraschall-Bildgeräten im Prinzipschaltbild, und

Fig. 2 die Erfindung im Prinzipschaltbild.

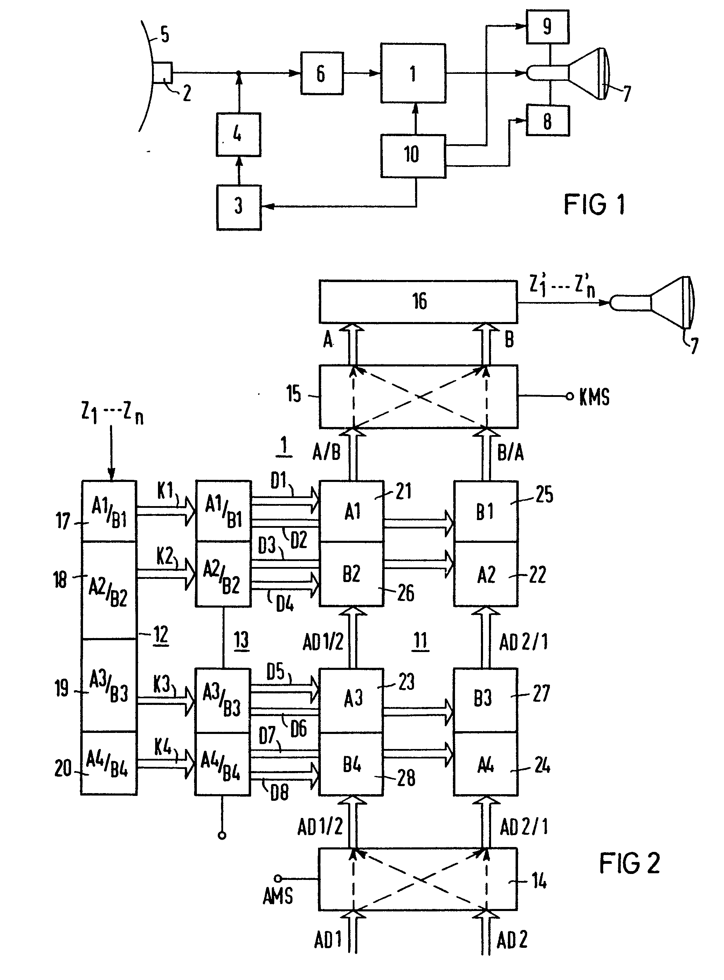

In der Fig. 1 ist die Vorrichtung zum Speichern der Ultraschallsignale als Block 1 dargestellt. Dieser Block 1 ist speziell Bestandteil eines Ultraschall-Bildgerätes. Dieses Bildgerät umfasst demnach in der üblichen Weise einen Ultraschallkopf 2 (z.B. mechanisch verschiebbarer oder schwenkbarer Wandler oder Ultraschall-Array), dem in Takt eines zentralen Taktgebers 3 Hochfrequenzimpulse eines HF-Impulssenders 4 zugeleitet werden. Der Schallkopf 2 strahlt daraufhin Ultraschall-Sendeimpulse in ein Untersuchungsobjekt 5 ab. Bei zeilenweiser Abtastung des Untersuchungsobjektes 5 (z.B. aufgrund mechanischer Verschiebung oder Schwenkung des Schallkopfes 2 oder aufgrund elektronischer Fortschaltung des Strahles entlang den Einzelelementen einer Wandlerelementanordnung wie beim Ultraschall-Array) werden die im Objekt 5 an unterschiedlichen internen Punkten reflektierten Ultraschall-Signale vom Ultraschallkopf 2 wieder empfangen und in elektrische Echosignale rückgewandelt. Die Echosignale werden in einem Empfangsverstärker 6 verstärkt und anschliessend in die Speichervorrichtung 1 ein- und wieder ausgelesen. Das Auslesen erfolgt zu einer Kathodenstrahlröhre 7 mit Zeilengenerator 8 und Bildgenerator 9 für den Aufbau eines Echosichtbildes. Die Gesamtsteuerung erfolgt mittels einer Steuereinrichtung 10.

Aufbau und Funktionsweise der Speichervorrichtung 1 werden nun im folgenden anhand des Prinzipschaltbildes der Fig. 2 näher erläutert.

In der Fig. 2 umfasst die Speichervorrichtung 1 einen Signal- oder Bildspeicher 11, dem eingangsseitig ein Zeilenspeicher 12 mit Register-Multiplexer 13 vorgeschaltet ist. Ausserdem sind dem Bildspeicher 11 ein Adressenmultiplexer 14 sowie ausgangsseitig ein Datenmultiplexer 15 mit Wechselpufferspeicher 16 zugeordnet.

Gemäss der Fig. 2 ist der Zeilenspeicher 12 in vier Abschnitte 17 bis 20 unterteilt. Bei seriellem Einlesen eines ersten und zweiten Ultraschallbildes A bzw. B wird jede Ultraschallzeile Z1 bis Zn also in insgesamt vier Signalabschnitte A1 bis A4 bzw. B1 bis B4 unterteilt. Diese werden durch paralleles Auslesen über Kanäle K1 bis K4 und über den Register-Multiplexer 13 unmittelbar als Parallelinformation über die Signaleingänge des Bildspeichers 11 (Datenkanäle D1 bis D8) in geeignet ausgewählte Speicherabschnitte 21 bis 28 eingeschrieben.

Im Ausführungsbeispiel der Fig. 2 ist der Bildspeicher 11 in 2 × 4 = 8 Speicherabschnitte 21 bis 28 unterteilt. Beispielsweise kann zur Realisierung in diesem Falle eine Speicher-Grundkarte mit 256 × 256 × 8 Bit gewählt werden. Die Teilkapazität aller Abschnittsadressen eines Speicherabschnitts 21 bis 28 würden somit 128 × 64 × 8 Bit betragen.

Im Ausführungsbeispiel der Fig. 2 sind Ein- und Auslesevorgang am Bildspeicher 11 so programmiert, dass am Eingang des Bildspeichers 11 anfallende Signale kreuzweise parallel ineinandergeschachtelt von einzelnen Speicherabschnitten übernommen werden. Die Auslesung dieser ineinandergeschalteten Speicherabschnitte erfolgt so, dass sich bei zeitlicher Aneinanderreihung der ausgelesenen Signalabschnitte das ursprüngliche Signal, d.h. im vorliegenden Fall das Ultraschallbild, von selbst ergibt.

Dieser Vorgang des kreuzweise verschachtelten Einlesens, der besonders hohe Einlesegeschwindigkeiten (gegenüber serieller Einlesung um den Faktor vier vergrösserte Geschwindigkeit) erlaubt, soll anhand eines Beispiels näher erläutert werden, welches speziell darauf ausgerichtet ist, wenigstens zwei Ultraschallbilder A und B, die zeitlich nacheinander anfallen, gleichzeitig nebeneinander oder übereinander am Bildschirm einer Oszillographenröhre 7 darzustellen.

Der Programmablauf des Einlesens und Wiederauslesens am Speicher 11 ist so, dass das in Signalabschnitten A1 bis A4 unterteilte erste Ultraschallbild A abschnittsweise parallel in die Speicherabschnitte 21 bis 24 eingeschrieben wird, was dort durch Einfügung der Bezeichnung A1 bis A4 näher gekennzeichnet ist. Entsprechend wird das unmittelbar darauf anfallende zweite Ultraschallbild B in Signalabschnitten B1 bis B4 unterteilt in die Speicherabschnitte 25 bis 28 des Speichers 11 überführt. Die betreffenden Speicherabschnitte 25 bis 28 sind hier mit B1 bis B4 gekennzeichnet. Man sieht an diesem Beispiel, dass die Einzelabschnitte A1 bis A4 des ersten Ultraschallbildes A mit den Einzelabschnitten B1 bis B4 des zweiten Ultraschallbildes B im Bildspeicher 11 kreuzweise in den Speicherabschnitten verschachtelt sind. Diese Verschachtelung erlaubt, wie schon vorstehend erwähnt, eine besonders rasche Einspeicherung von Ultraschallbildern in den Bildspeicher 11.

Das Auslesen der kreuzweise verschachtelt eingeschriebenen Signalabschnitte A1 bis A4 und B1 bis B4 erfolgt gesteuert vom Adressenmultiplexer 14 (Eingangsadressen AD1, AD2; Ausgangsadressen AD1/2, AD2/1; Adress-Multiplex-Steuersignal AMS) in der Weise, dass in zeitlicher Aufeinanderfolge über den Daten-Multiplexer 15 die Signalabschnitte A1, B2, A3, B4 und jeweils parallel dazu die Signalabschnitte B1, A2, B3, A4 ausgelesen werden. Der Daten-Multiplexer 15 vertauscht dabei B2 mit A2 und B4 mit A4, wie durch die gestrichelten Pfeile im Multiplexer 15 angedeutet ist. Die beiden Bilder A und B sind dann wieder richtig zusammengesetzt, wie durch die beiden separaten Pfeilausgänge A, B des Kanal-Multiplexers 15 angedeutet ist. Die beiden Bilder A und B werden parallel in den Pufferspeicher 16 eingelesen. Sie werden hier nach serieller zeilenweiser Auslesung aus dem Pufferspeicher 16 (Video-Zeilen Z1' bis Zn') auf ein und derselben Oszillograpahenröhre 7 direkt nebeneinander abgebildet.

Es versteht sich von selbst, dass durch andersartige Programmierung der Ablaufsteuerung auch ein Ineinander- oder Übereinanderschreiben der beiden Bilder A und B möglich ist. Ebensogut kann der Ablauf auch so programmiert sein, dass mehr als zwei Bilder A und B eingespeichert und gemeinsam am Oszillographen 7 wieder ausgelesen werden. Selbstverständlich ist auch die Umschaltung auf nur ein einziges Bild möglich. Dabei kann ein einzelnes gespeichertes Bild auch beispielsweise mit einem real-time-Bild zusammen dargestellt werden. Der Einlesevorgang in den Bildspeicher 11 kann jedoch auch so programmiert sein, dass beispielsweise bei Bedarf zeitweise von Parallelübernahme von Signalabschnitten in den Signalspeicher 11 auf serielle Übernahme umgeschaltet wird. Hierdurch verzögert sich zwar der Einlesevorgang. Dafür besteht jedoch die Möglichkeit, Ultraschallbilder unterschiedlicher Art darzustellen. Beispielsweise kann ein B-Bild mit einem TM-Bild dargestellt werden.

Das beschriebene Ausführungsbeispiel eignet sich besonders gut für den Einsatz bei Parallel-Abtastung von Objekten. Für den Fall nichtparalleler Abtastung, wie z.B. Sektor-, Trapez-, Compound-Abtastung oder dgl., ergibt sich ebenfalls optimaler Einsatz, wenn der Speicher 11 in einfacher Weise hinsichtlich der Adressierung in Anpassung an den geänderten Anwendungsfall umorganisiert wird.

## Patentansprüche

1. Vorrichtung zum Speichern von digitalen elektrischen Signalen, die in Signalzeilen aufeinanderfolgen, insbesondere von Ultraschallsignalen, mit einem Signalspeicher mit Signaleingängen zum Einspeichern der Signale und Signalausgängen zum Auslesen der gespeicherten Signale, vorzugsweise zum Auslesen in ein Aufzeichnungsgerät, wie ein Bildgerät, wobei der Signalspeicher in eine Anzahl von Speicherabschnitten unterteilt ist, dadurch gekennzeichnet, dass ein Zeilenspeicher (12) vorgesehen ist, in den die Signalzeilen (A, B) seriell eingelesen werden, in dem jede Signalzeile (A, B) in Signalabschnitte (A1 bis A4, B1 bis B4) aufgeteilt wird und aus dem diese Signalabschnitte parallel zueinander über einen Registermultiplexer (13) zum Signalspeicher (11) ausgelesen werden, so dass die an den Signaleingängen (D1 bis D8) des Signalspeichers (11) anfallenden Signale in die erwähnten Signalabschnitte (A1 bis A4, B1 bis B4) aufgeteilt sind, dass die zeitlich nacheinander angefallenen Signalabschnitte (A1 bis A4, B1 bis B4) jeweils in Parallelformation in den Speicherabschnitten (21 bis 28) des Signalspeichers (11) einspeicherbar sind, wobei die Daten jedes Signalabschnitts (A1 bis A4, B1 bis B4) seriell in den zugeordneten Speicherabschnitt eingelesen werden, und dass die Speicherabschnitte (21 bis 28) zeitlich nacheinander orthogonal auslesbar sind in einer solchen Reihenfolge, dass sich nach Zusammensetzung wieder die aus den Signalabschnitten in richtiger Reihenfolge zusammengesetzten Signalzeilen ergeben.

2. Vorrichtung nach Anspruch 1, dadurch gekennzeichnet, dass die Speicherabschnitte (21 bis 28) im Signalspeicher (11) für zeitlich aufeinanderfolgende Signale (A, B) kreuzweise parallel ineinandergeschachtelt sind in der Weise, dass bei der Orthogonal-Auslesung entsprechend der Reihenfolge des Anfallens von Signalabschnitten (A1 bis A4; B1 bis B4) und bei der Zusammensetzung deren zeitgerechte Aneinanderreihung zu den Formen der Ursprungssignale (A, B) von selbst erfolgt.

3. Vorrichtung nach Anspruch 1 oder 2, dadurch gekennzeichnet, dass der Signalspeicher (11) in vier erste Speicherabschnitte (21 bis 24), in die ein erstes Signal (A) einspeicherbar ist, und vier weitere Speicherabschnitte (25 bis 28), in die ein zweites Signal (B) einspeicherbar ist, unterteilt ist, wobei die ersten und zweiten Speicherabschnitte (21 bis 24, 25 bis 28) kreuzweise von einem Adressenmultiplexer (14) ansteuerbar sind.

4. Vorrichtung nach einem der Ansprüche 1 bis 3, dadurch gekennzeichnet, dass das Auslesen und Zusammensetzen der Signalabschnitte (A1 bis A4, B1 bis B4) über einen Datenmultiplexer (15) mit anschliessendem Wechselpufferspeicher (16) erfolgt.

5. Vorrichtung nach einem der Ansprüche 1 bis 4, dadurch gekennzeichnet, dass das Einschreiben und Auslesen der Signalabschnitte (A1 bis A4, B1 bis B4) in den bzw. aus dem Signalspeicher (11) in Speicherabschnitte (21 bis 28) mit programmierbarer Formation erfolgt.

6. Vorrichtung nach Anspruch 5, dadurch gekennzeichnet, dass der Einlesevorgang in den Signalspeicher (11) so programmiert ist, dass zeitweise von Parallelübernahme von Signalabschnitten (A1 bis A4, B1 bis B4) auf serielle Übernahme umgeschaltet werden kann.

7. Vorrichtung nach Anspruch 5 oder 6, dadurch gekennzeichnet, dass der Auslesevorgang aus dem Signalspeicher (11) so programmierbar ist, dass bestimmte Signalabschnitte zu voneinander unterschiedlichen Darstellungsmodes auslesbar sind.

8. Vorrichtung nach Anspruch 7, dadurch gekennzeichnet, dass unterschiedliche Signale oder Signalformen in Signalabschnitten im Signalspeicher (11) speicherbar sind, die einzeln oder in Kombination miteinander im gleichen Darstellungsmode oder in unterschiedlichen Darstellungsmodes auslesbar sind.

9. Vorrichtung nach einem der Ansprüche 1 bis 8, dadurch gekennzeichnet, dass der Signalspeicher (11) ein Ultraschall-Speicher ist, der so programmierbar ist, dass ein einzelnes Ultraschallbild oder mehrere Ultraschallbilder (A, B) einspeicherbar sind.

10. Vorrichtung nach Anspruch 9, dadurch gekennzeichnet, dass bei mehreren Ultraschallbildern (A, B) die Auslesung nach Programm so erfolgt, dass die Ultraschallbilder in beliebiger Konfiguration zueinander gleichzeitig an einem Bildgerät (7), insbesondere auf einer Kathodenstrahlröhre, darstellbar sind.

11. Vorrichtung nach Anspruch 10, dadurch gekennzeichnet, dass wenigstens zwei Ultraschallbilder neben- oder übereinander darstellbar sind.

12. Vorrichtung nach Anspruch 10 oder 11, dadurch gekennzeichnet, dass bei Darstellung von wenigstens zwei Bildern eines ein gespeichertes Bild und das andere ein real-time-Bild ist.

13. Vorrichtung nach einem der Ansprüche 9 bis 12, dadurch gekennzeichnet, dass wenigstens ein B-Bild mit einem TM-Bild gleichzeitig darstellbar ist.

## Claims

1. Device for storing digital electrical signals which follow one another in signal rows, in particular ultrasonic signals, with a signal store having signal inputs for the input of the signals and signal outputs for the read-out of the stored signals, preferably for read-out into a recording device such as a video device, where the signal store is divided into a number of store sections, characterised in that a row store (12) is provided into which the signal rows (A, B) are input in serial form, and in which each signal row (A, B) is devided into signal sections (A1 to A4, B1 to B4), and from which these signal sections are read-out in parallel to the signal store (11) via a register multiplexer (13), so that the signals which occur at the signal inputs (D1 to D8) of the signal store (11) are divided into the aforementioned signal sections (A1 to A4, B1 to B4), that the signal sections (A1 to A4, B1 to B4) which occur consecutively in time can each be input in parallel formation into the store sections (21 to 28) of the signal store (11), where the items of data of each signal section (A1 to A4, B1 to B4) are input in serial form into the allocated store section, and that the store sections (21 to 28) can be orthogonally read-out consecutively in time in a sequence which ensures that following assembly the signal rows are composed of the signal sections in the correct sequence.

2. Device as claimed in claim 1, characterised in that the store sections (21 to 28) in the signal store (11) for signals (A, B) which are consecutive in time are cross-wise telescoped in parallel in such manner that during the orthogonal read-out in accordance with the sequence of the occurence of signal sections (A1 to A4; B1 to B4) and during the assembly, they are automatically promptly lined up so as to adopt the formations of the original signals (A, B).

3. Device as claimed in claim 1 or 2, characterised in that the signal store (11) can be divided into four first store sections (21 to 24) into which a first signal (A) can be input, and four further store sections (25 to 28) into which a second signal (B) can be input, where the first and second store sections (21 to 24, 25 to 28) can be driven cross-wise by an address multiplexer (14).

4. Device as claimed in one of the claims 1 to 3, characterised in that the read-out and assembly of the signal sections (A1 to A4, B1 to B4) is effected by a data multiplexer (15) adjoined by an alternating buffer store (16).

5. Device as claimed in one of the claims 1 to 4, characterised in that the input and read-out of the signal sections (A1 to A4, B1 to B4) into and out of the signal store (11) is effected in store sections (21 to 28) with programmable formation.

6. Device as claimed in claim 5, characterised in that the input process into the signal store (11) is programmed to be such that periodically it is possible to switch over from parallel transfer of signal sections (A1 to A4, B1 to B4) to serial transfer.

7. Device as claimed in claim 5 or 6, characterised in that the read-out process from the signal store (11) is programmed to be such that specific signal sections can be read-out in representation modes which differ from one another.

8. Device as claimed in claim 7, characterised in that different signals or signal forms can be stored in signal sections within the signal store (11) which can be read-out individually or in combination with one another in the same representation mode or in different representation modes.

9. Device as claimed in one of the claims 1 to 8, characterised in that the signal store (11) is an ultrasonic store which can be programmed in such manner that one signal ultrasonic image or a plurality of ultrasonic images (A, B) can be input.

10. Device as claimed in claim 9, characterised in that in the event of a plurality of ultrasonic images (A, B) the programmed read-out is effected in such manner that the ultrasomic images can be represented in an arbitrary configuration relative to one another simultaneously on a video device (7), in particular a cathode ray tube.

10. Device as claimed in claim 10, characterised in that at least two ultrasonic images can be represented one beside another or one above another.

12. Device as claimed in claim 10 or 11, characterised in that when at least two images are represented, one is a stored image and the other is a real-time image.

13. Device as claimed in one of the claims 9 to 12, characterised in that at least one B-image can be represented simultaneously to a TM-image.

**Revendications**

1. Dispositif pour mémoriser des signaux électriques numériques qui se succèdent suivant des lignes de signaux, en particulier des signaux ultrasonores, avec une mémoire de signaux possédant des entrées de signaux pour introduire les signaux et des sorties de signaux pour la lecture des signaux mémorisés, de préférence pour leur introduction danns un appareil d'enregistrement, tel qu'un appareil à représentation d'images, les mémoires de signaux étant subdivisées en un nombre de zones de mémoire, caractérisé par le fait qu'il est prévu une mémoire de lignes (12) dans laquelle sont introduites de façon sérielle les lignes de signaux (A, B) par subdivision de chaque ligne de signaux (A, B) en sections de signaux et à partir de laquelle ces sections de signaux (13) sont transférées à la mémoire de signaux (11) parallèlement entre elles et par l'intermédiaire d'un multiplexeur de registres (13), de manière que les signaux qui se présentent aux entrées de signaux (D1 à D8) de la mémoire de signaux (11) sont subdivisés en les sections de signaux A1 à A4, B1 à B4) sus-mentionnées, que les sections de signaux (A1 à A4, B1 à B4) qui se présentent successivement dans le temps sont susceptibles d'être respectivement mémorisées en formation parallèle dans les zones de mémoire (21 à 28) de la mémoire de signaux (11), les données de chaque section de signaux (A1 à A4, B1 à B4) étant inscrites sériellement dans la zone de mémoire associée, et que les zones de mémoire (21 à 28) sont susceptibles d'être lues orthogonalement et successivement dans le temps dans un ordre tel qu'il en résulte, après assemblage, à nouveau les lignes de signaux composées dans l'ordre correct à partir des sections de signaux.

2. Dispositif selon la revendication 1, caractérisé par le fait que les zones de mémoire (21 à 28) sont, dans la mémoire de signaux (11), pour les signaux (A, B) qui se succèdent dans le temps, imbriquées en parallèle et de façon croisée, et cela de telle manière que lors de la lecture orthogonale selon l'ordre des sections de signaux qui se présentent (A1 à A4; B1 à B4) et de la composition, leur assemblage correct dans le temps, s'opère automatiquement pour donner les formes de signaux d'origine (A, B).

3. Dispositif selon la revendication 1 ou 2, caractérisé par le fait que la mémoire de signaux (11) est subdivisée en quatre premières zones de mémoire (21 à 24) dans lesquelles est susceptible d'être mémorisé un premier signal (A) et en quatre autres zones de mémoire (25 à 28) dans lesquelles est susceptible d'être mémorisé un second signal (B), les premières et secondes zones de mémoire (21 à 24, 25 à 28) étant susceptibles d'être attaquées de façon croisée par un multiplexeur d'adresses (14).

4. Dispositif selon l'une des revendications 1 à 3, caractérisé par le fait que la lecture et l'assemblage des sections de signaux (A1 à A4, B1 à B4) ont lieu par l'intermédiaire d'un multiplexeur de données avec mémoire-tampon (16) qui y fait suite.

5. Dispositif selon l'une des revendications 1 à 4, caractérisé par le fait que l'inscription et la lecture des sections de signaux (A1 à A4, B1 à B4) dans ou à partir de leur mémoire de signaux (11), ont lieu dans des zones de mémoire (21 à 28) avec formation susceptible d'être programmée.

6. Dispositif selon la revendication 5, caractérisé par le fait que l'opération d'inscription dans la mémoire de signaux (11) est programmée de telle façon que de temps en temps on peut commuter de la prise en charge parallèle de section de signaux (A1 à A4, B1 à B4) à une prise en charge sérielle.

7. Dispositif selon la revendication 5 ou 6, caractérisé par le fait que l'opération de lecture à partir de la mémoire de signaux (11) est susceptible d'être programmée de telle façon que certaines sections de signaux sont susceptibles d'être lues suivant des modes de représentation qui diffèrent entre eux.

8. Dispositif selon la revendication 7, caractérisé par le fait que des signaux ou des formes de signaux différents sont susceptibles d'être mémorisés suivant des sections de signaux danns la mémoire de signaux (11) et susceptibles d'être lues séparément ou en combinaison entre elles dans le même mode de représentation ou dans des modes de représentation différents.

9. Dispositif selon l'une des revendications 1 à 8, caractérisé par le fait que la mémoire de signaux (11) est une mémoire à ultrasonic, qui est susceptible d'être programmée de telle façon que les images ultrasonores individuelles ou plusieurs images ultrasonores (A, B) sont susceptibles d'être mémorisées.

10. Dispositif selon la revendication 9, caractérisé par le fait que dans le cas de plusieurs images ultrasonores (A, B) la lecture suivant un programme a lieu de telle façon que les images ultrasonores sont susceptibles d'être représentées suivant une configuration quelconque simultanément sur un appareil de visualisation (7), en particulier sur un tube à rayon cathodique.

11. Dispositif selon la revendication 10, caractérisé par le fait qu'au moins deux images ultrasonores sont susceptibles d'être représentées l'une à côté de l'autre ou l'une au-dessus de l'autre.

12. Dispositif selon la revendication 10 ou 11, caractérisé par le fait que dans le cas de la représentation d'au moins deux images, l'une est une image mémorisée et l'autre est une image en temps réel.

13. Dispositif selon l'une des revendications 9 à 12, caractérisé par le fait qu'au moins une image B est susceptible d'être représentée en même temps qu'une image TM.

FIG 1

FIG 2